Europäisches Patentamt

European Patent Office

Office européen des brevets

(19)

(11) **EP 0 737 483 A1**

(12) **EUROPEAN PATENT APPLICATION**

(43) Date of publication:
16.10.1996 Bulletin 1996/42

(51) Int. Cl.⁶: **A61M 5/142**

(21) Application number: 96101684.7

(22) Date of filing: 06.02.1996

(84) Designated Contracting States:
DE FR GB

(30) Priority: 12.04.1995 SE 9501364

(71) Applicant: Siemens Elema AB
171 95 Solna 1 (SE)

(72) Inventors:
• Sjöholm, Gösta
  S-178 34 Ekerö (SE)
• Slettenmark, Bruno
  S-175 60 Järfälla (SE)

(54) **Pump**

(57)     A pump for viscous substances comprises a pump chamber (5) with an inlet (10), intended for connection to a source of the substance to be pumped, and an outlet (12), equipped with a valve, for pumping out the substance sucked into the pump chamber during an intake phase. The pumping action is achieved by varying the volume of the pump chamber. A unit (8) is arranged to control the variation of the volume of the pump chamber, such that the delivery phase is considerably shorter than the intake phase. The inlet is further devised such that it presents a greater resistance to flow than the outlet when the outlet valve (14) is open, so the backflow of substance through the inlet during the delivery phase only corresponds to a minor portion of the stroke volume of the pump.

## Description

The present invention relates to a pump for viscous substances comprising a pump chamber with an inlet, intended for connection to a source of the substance to be pumped, and an outlet, equipped with a valve, for pumping out the substance, sucked into the pump chamber during an intake phase, pumping action being achieved by varying the volume of the pump chamber.

A pump of this kind is previously known through DE,A1,33 20 443. Such so-called micropumps are intended for pumping small, exact flows of viscous substances, such as liquid medication in implantable medication pumps.

For safety reasons, the medication reservoir in certain kinds of medication pumps is kept at a sub-ambient pressure, see US,A,4 191 181. The pump must then be capable of pumping liquid from this lower pressure into the pump chamber, which has appeared to be difficult with pumps manufactured by so-called micromechanical technics.

The current type of pumps must also have reliable valve functions and be able to pump gas bubbles (air) out of the low pressure reservoir. These requirements can be achieved with a pump according to US,A,4 808 089. The disadvantage of these pumps is that they are expensive to manufacture, since a plurality of parts to be assembled must be manufactured with very close tolerances.

The purpose of the present invention is to eliminate these disadvantages of prior art pumps and simplify the construction, so the pumps can be mass manufactured at low cost, by efficient methods, with simultaneous retention of their ability to pump very small flows with great accuracy and improved operating reliability.

This purpose is achieved with a pump of the kind stated in the introduction with the characteristics set forth in claim 1.

With the pump according to the invention, the need for an inlet valve is eliminated, thereby increasing operating reliability. The pump can suitably be made of silicon using etching methods. The pump can then be made very small with a very small chamber volume. Further, the pump can advantageously be constructed, so its dead space is substantially equal to zero when the pump in its operation cycle is in a position at which the pump chamber has the smallest volume, and very small volumes can be pumped with great accuracy.

According to advantageous embodiments of the pump according to the invention, the increased flow resistance in the inlet can be realized with a restriction or constriction in the inlet, e.g. by designing the inlet as a relatively long and narrow channel, possibly meander shaped, to reduce the space requirements, or as a capillary. This reduces accordingly the backflow of substance during the pump's compression or delivery phase. The inlet can advantageously be devised so the backflow of substance through the inlet during the delivery phase is less than 5% of the stroke volume of the pump.

The pumping function is achieved by varying the volume of the pump chamber and according to advantageous embodiments of the pump according to the invention, one wall of the pump chamber is moveable, and the volume of the pump chamber can be varied by moving said wall, alternatively one wall of the pump chamber comprises a mechanically, pneumatically, hydraulically, electromagnetically, piezoelectrically, magnetostrictively or electrostatically actuatable membrane for varying the volume of the pump chamber.

According to other advantageous embodiments of the pump according to the invention, the outlet valve is arranged to be opened by the pressure difference produced across the valve during the pump stroke or the delivery phase, alternatively the outlet valve can be devised with a separate, maneuverable opening mechanism.

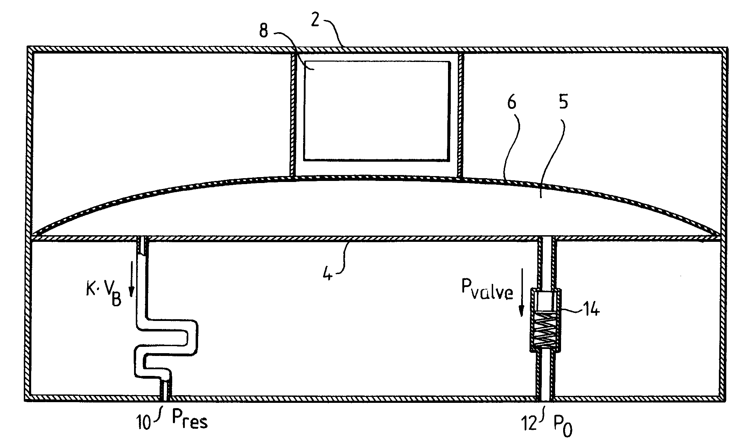

An exemplifying embodiment of the pump according to the invention will now be described in greater detail, with reference to the enclosed drawing on which a cross-section of an embodiment of the pump chosen as an example is shown.

The embodiment of the pump according to the invention, as shown in the figure, is contained within an enclosure 2. The pump chamber 5 is limited by the wall 4 and the membrane 6.

The membrane 6 is maneuverable with a membrane activator 8. This activator can consist of e.g. a piezoelectric body, a bimorphous piezoelectric structure etc., which is lengthened and deformed respectively when an electrical voltage is applied to the body, and which is arranged in a guide, such that it then presses the membrane 6 towards the wall 4 in order to produce the compression or delivery phase, i.e. to reduce the volume of the pump chamber 5.

However, the membrane activator 8 can also be of another type which actuates the membrane 6 mechanically, pneumatically, hydraulicly, electromagneticly, magnetostrictively or electrostatically.

The membrane itself can also be devised as a bimorphous plate which performs the desired movement when excited.

An inlet 10, e.g. in the form of a relatively long channel, or capillary, depicted as a meandering, narrow channel in the figure, leads to the pump chamber 5. The channel, or the capillary, is made of a material which all the time remains coated with a film of the pumped substance, i.e. it is wetted by the substance. The pump chamber 5 has an outlet 12, equipped with a one-way valve 14.

The pump operates according to the following cycle:

The starting position of the pump is the position shown in the figure with a maximum pump chamber volume. The pump is activated to execute the pumping stroke, causing the membrane 6 to be pushed downwards in the figure towards the wall 4 by the membrane activator 8, so the volume of the chamber is reduced. The outlet valve 14 opens, either because of the pressure gradient produced across the valve or because of some active operation by a separate, maneuverable opening mechanism (not shown in the figure).

The inlet 10 is dimensioned such that the backflow through it during this compression or delivery phase is small compared to the stroke volume of the pump, typically less than 5% of thereof, see below. When this delivery phase, or pumping stroke, which is relatively rapid, has been completed, the expansion or intake phase begins, during which the chamber volume 5 increases. The outlet valve 14 then closes, and pump substance is sucked in through the inlet 10. The intake phase takes much longer time than the pump stroke or the delivery phase, so there is time enough for filling the pump chamber 5 without cavitation before the next pump stroke, despite the small flow in the inlet 10.

So if $P_{Omax}$ designates the highest ambient pressure against which the pump must be able to pump, and $P_{valve}$ designates the pressure fall across the outlet valve 14 during the pump stroke or delivery phase, which pressure fall is assumed to be constant during the delivery phase, the maximum pressure in the pump chamber 5 becomes

$$P \approx P_{Omax} + P_{valve}$$

If the maximum volume of backflow through the inlet during a pump stroke shall be less than $k \cdot V_B$, where k designates a factor < 1 determining the largest amount of permissible liquid backflow $k \cdot V_B$ through the inlet during the pump stroke, and $V_B$ designates the chamber volume of the pump, which is assumed to be approximately equal to the stroke volume of the pump, i.e. dead space is negligible, the following inequality must be fulfilled:

$$\frac{(P_{Omax} + P_{valve} - P_{res}) \cdot \pi \cdot r^4 \cdot T}{8 \cdot \eta \cdot \ell} < k \cdot V_B$$

in which $P_{res}$ designates the pressure in the reservoir for the substance to be pumped on the intake side of the pump, r is the radius of the inlet, T is the duration of the d pump's elivery phase, $\eta$ is the viscosity of the pumped substance at the prevailing temperature and $\ell$ is the length of the pump's inlet.

Here, the mass forces exerted by the substance in the inlet have been neglected but they will have a favorable effect, i.e. the backflow through the inlet will actually be less than the above inequality indicates.

If the pump is to be capable of pumping air, e.g. an air bubble from an area with lower pressure on the inlet side than on the outlet side, the pump must be constructed such that the dead space is substantially equal to zero when the pump is in the position during its operating cycle in which its chamber has smallest volume.

If an air bubble with a diameter larger than the diameter of the inlet channel or the capillary were to block the inlet, the pump must be capable of overcoming the capillary force and suck in air into the pump chamber during the intake or expansion phase to avoid that the pump stops working. Therefore, the following relation must be fulfilled:

$$\frac{2 \cdot \pi \cdot r \cdot \sigma \cdot \cos(\phi)}{\pi \cdot r^2} < P_{res} - P_{vapor} \qquad (1)$$

i.e.

$$r > \frac{2 \cdot \sigma \cdot \cos(\phi)}{P_{res} - P_{vapor}} \qquad (2)$$

where $\sigma$ designates the surface tension of the pumped substance, $\phi$ is the contact angle of the pumped substance in the inlet channel, or the capillary, in the presence of air and $V_{vapor}$ designates the vapor pressure of the pumped substance at the prevailing temperature.

By supplying the pump according to the invention with an air blocking device according to EP,A1,0 586 740, the accumulation of large amounts of air in front of the inlet is avoided. This air blocking device prevents the passage of e.g. air bubbles with a diameter larger than 100 $\mu$m. The volume of such an air bubble is only $5 \times 10^{-4}$ $\mu$l, i.e.$<< V_B$ and $<<$ volume of the inlet channel, or inlet capillary. The inlet channel, or inlet capillary, and the pump chamber can accordingly only be filled with air to a fraction of their volume. If the inlet capillary and pump chamber should be filled with air, there is a risk that the pumping effect is stopped, i.e. air oscillates back and forth through the capillary.

If it is assumed that there is a certain amount of air in the pump chamber where the inlet capillary discharges, the air will flow back into the capillary at every pump stroke, pushing a column of liquid, whose length declines, ahead of it. To ensure that the pump operates reliably and is able to pump air out through the outlet valve, the air column must not be allowed to pass the inlet in the reverse direction and enter the reservoir holding the substance to be pumped during the delivery or compression phase. Otherwise there is a risk that the same amount of air will oscillate back and forth between the pump chamber and the reservoir with a permanent reduction in output volume as a result.

If the time it takes for the column of air to reach the reservoir holding the substance to be pumped is designated by $T_1$, the following applies:

$$T_1 = \frac{8 \cdot \eta \cdot \ell^2}{(P_{Omax} + P_{valve} - P_{cap} - P_{res}) \cdot 2 \cdot r^2}$$

in which

$$P_{cap} = \frac{2 \cdot \sigma \cdot \cos(\phi)}{r}$$

is the pressure required to overcome the capillary force in the inlet capillary at the liquid/air meniscus.

If the pump is to be capable of pumping air out of the pump chamber, the condition $T_1 > T$ must be fulfilled, where T designates the duration of the compression phase of the pump.

The following inequality is derived therefrom:

$$\frac{8 \cdot \eta \cdot \ell^2}{(P_{Omax}+P_{valve}-P_{cap}-P_{res}) \cdot 2 \cdot r^2} > T \qquad (3)$$

We have then not considered the mass forces on the column of liquid in the inlet capillary, but they have a favorable effect, i.e. $T_1$ is greater in reality than stated above.

If the shortest time required to refill the pump chamber is denoted by $T_{2min}$, the following relation is valid

$$T_{2min} = \frac{V_B \cdot 8 \cdot \ell \cdot \eta}{(P_{res}-P_{vapor}) \cdot \pi \cdot r^4}$$

The maximum pumping rate is given by:

$$f_{max} = \frac{1}{T_{2min}+T}$$

And the maximum pump flow is

$$Q_{max} = f_{max} \cdot V_B$$

Mass forces have also been neglected in the calculation of $T_{2min}$, so $Q_{max}$ is less than what is stated above.

In order to obtain a pump with the desired properties, it must be dimensioned such that the inequalities 1-3 above are fulfilled at the same time.

For a pump which is to pump medication in aqueous solution at 37° C with the following parameters

| | |
|---|---|
| $P_{Omax}$ | $= 1.2 \cdot 10^5$ Pa |
| $P_{res}$ | $= 0.7 \cdot 10^5$ Pa |
| $P_{vapor}$ | $= 63 \cdot 10^2$ Pa |
| $P_{valve}$ | $= 0.1 \cdot 10^5$ Pa |
| $V_B$ | $= 1 \cdot 10^{-10}$ m$^3$ |
| k | $= 0.05$ |
| $\eta$ | $= 0.7 \cdot 10^{-3}$ Ns/m$^2$ |
| $\sigma$ | $= 50 \cdot 10^{-3}$ N/m |
| $\phi$ | $= 0$ degrees |
| T | $= 10^{-4}$ s |

$\ell = 5 \cdot 10^{-3}$ m and r $= 50 \cdot 10^{-6}$ can be selected, all conditions then being met and $Q_{max}$ then amounting to about 43 μl/s.

The pump according to the invention as described above can be advantageously manufactured of silicon and made very small, by means of micromechanical technics or etching procedures, and with a very small chamber volume. Moreover, manufacturing costs can be kept low. The pump is further suitable for pumping very small flows with great accuracy, and is therefore suitable for use in medical implants.

**Claims**

1. A pump for viscous substances comprising a pump chamber with an inlet, intended for connection to a source of the substance to be pumped, and an outlet, equipped with a valve, for pumping out the substance sucked into the pump chamber during an intake phase, pumping action being achieved by varying the volume of the pump chamber, **characterized in** that an unit is arranged to control the variation of the volume of the pump chamber, such that the delivery phase is considerably shorter than the intake phase, and in that the inlet is so devised that it presents a greater resistance to flow than the outlet when the outlet valve is open, so the backflow of substance through the inlet during the delivery phase only corresponds to a fraction of the stroke volume of the pump.

2. The pump according to claim 1, **characterized in** that the inlet is devised with a restriction or constriction in order to achieve said increased resistance to flow.

3. The pump according to claim 1, **characterized in** that the inlet is devised as a relatively long and narrow channel or capillary, in order to achieve said increased resistance to flow.

4. The pump according to claim 3, the inlet being devised as a relatively long and narrow channel, **characterized in** that the said channel is meander shaped.

5. The pump according to claim 3, the inlet being devised as a capillary, **characterized in** that the capillary is made of a material which always remains coated with a film of the pumped substance.

6. The pump according to any one of the claims 1 - 5, **characterized in** that the inlet is so devised that the backflow of substance through the inlet during the delivery phase is less than 5% of the stroke volume of the pump.

7. The pump according to any one of the claims 1 - 6, **characterized in** that one wall of the pump chamber is movable, and in that the volume of the pump chamber can be varied by moving said wall.

8. The pump according to any one of the claims 1 - 6, **characterized in** that one wall of the pump chamber comprises a mechanically, pneumatically, hydraulically, electromagnetically, piezoelectrically, magnetostrictively or electrostatically actuatable membrane for varying the volume of the pump chamber.

9. The pump according to any one of the claims 1 - 6, **characterized in** that one wall of the pump chamber comprises a bimorphous plate, arranged to perform, when activated, the movement required for varying the volume of the pump chamber.

10. The pump according to any one of the claims 1 - 9, **characterized in** that the outlet valve is arranged to be opened by the pressure difference produced across the valve during the delivery phase.

11. The pump according to any one of the claims 1 - 9, **characterized in** that the outlet valve is devised with a separate, maneuverable opening mechanism.

12. The pump according to any of claims 1 - 11, **characterized in** that it is made of silicon.

$K \cdot V_B$

$P_{valve}$

8 2

6 5

4

14

10 $P_{res}$

12 $P_0$

EP 0 737 483 A1

| | European Patent Office | **EUROPEAN SEARCH REPORT** | Application Number EP 96 10 1684.7 Page 1 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.6) |
|---|---|---|---|
| A | DE, A1, 3320443 (SIEMENS AG), 6 December 1984 (06.12.84) * figure 1, abstract * ----- | 1-12 | A61M 5/142 |
| | | | **TECHNICAL FIELDS SEARCHED (Int. Cl.6)** |
| | | | A61M F04B |

| The present search report has been drawn up for all claims | | |
|---|---|---|
| Place of search STOCKHOLM | Date of completion of the search 26 June 1996 | Examiner MAY HALLNE |

CATEGORY OF CITED DOCUMENTS

X : particularly relevant if taken alone
Y : particularly relevant if combined with another
    document of the same category
A : technological background
O : non-written disclosure
P : intermidiate document

T : theory or principle underlying the invention
E : earlier patent document, but published on, or
    after the filing date
D : document cited in the application
L : document cited for other reasons
..........................................
& : member of the same patent family, corresponding
    document

EPO FORM 1503 03.82 (P0401)

7